# EUROPEAN PATENT SPECIFICATION

(11) **EP 0 582 153 B1**
(45) Date of publication and mention of the grant of the patent: **17.01.1996**
(21) Application number: 93111844.2
(22) Date of filing: 23.07.1993
(51) Int. Cl.: C07H 3/02

(54) **Method of preparing 4-deoxy-D-mannose**
Verfahren zur Herstellung von 4-Deoxy-D-Mannose
Procédé de préparation de 4-déoxy-D-mannose

(30) Priority: 23.07.1992 JP 197006/92
(43) Date of publication of application: 09.02.1994
(73) Proprietor: Japan Tobacco Inc., Shinagawa-ku, Tokyo 140 (JP)
(72) Inventor: Matsumoto, Katsuya, c/o JAPAN TOBACCO INC., Yokohama-shi, Kanagawa-ken 227 (JP); Ebata, Takashi, c/o JAPAN TOBACCO INC., Yokohama-shi, Kanagawa-ken 227 (JP); Koseki, Koshi, c/o JAPAN TOBACCO INC., Yokohama-shi, Kanagawa-ken 227 (JP); Okano, Koji, c/o JAPAN TOBACCO INC., Yokohama-shi, Kanagawa-ken 227 (JP); Kawakami, Hiroshi, c/o JAPAN TOBACCO INC., Yokohama-shi, Kanagawa-ken 227 (JP); Matsushita, Hajime, c/o JAPAN TOBACCO INC., Yokohama-shi, Kanagawa-ken 227 (JP)
(74) Representative: Reinhard - Skuhra - Weise & Partner

(56) References cited:
- COLL. CZECH. CHEM. COMMUN. vol. 37 , 1972 pages 3632 - 3639 T. TRNKA ET AL. 'Synthesis with Anhydro Sugars. XIII.'
- COLL. CZECH. CHEM. COMMUN. vol. 48 , 1983 pages 2386 - 2394 I. CERNY ET AL 'Preparation of 2-amino-2,4-dideoxy-D-lyxo-hexopyranose (4-Deoxy-D-mannosamine) from 1,6-Anhydro-beta-D-glucopyranose'
- J. CHEM. SOC., PERKIN I, 1977 pages 1157 - 1162 R.C. CAMBIE ET AL. 'Iodoacetoxylation of 3-t-Butylcyclohexene'
- CHEM. BER. vol. 109 , 1976 pages 337 - 344 P. KÖLL ET AL. 'Synthese der isomeren Enone aus der Reihe der 1,6-Anhydro-beta-D-hexopyranosen'

## Description

The present invention relates to a method of preparing 4-deoxy-D-mannose.

In recent year, chemical compounds containing natural occurring sugar and sugar-analog compounds have been drawn attention as useful physiologically active ingredients in a finechemical field such as pharmaceutical agents and agricultural agents. Many studies have been widely conducted on synthesis of the above chemical compounds.

A widely available sugar or a sugar derivative such as D-glucose, D-mannose and D-galactose is known as a monosaccharide which can be a constitutional unit of the above-mentioned sugar compounds. Development of a sugar compound using a rare sugar except the above-mentioned general sugar as a constitutional unit is a matter of a global importance from the view point of improving a function of a sugar chain and adding a novel function thereto. Furthermore, in synthesizing a useful sugar compound having a novel function, it is effective to use the rare sugar as a starting raw material. From the above-mentioned view-point, to develop a convenient method of preparing a rare sugar in a large scale is considered to be important.

4-deoxy-D-mannose represented by the following formula (1) is a kind of the above-mentioned rare sugar compound and an effective compound used as a composition unit of various sugar compounds or a starting raw material for synthesizing thereof.

Formula (1)

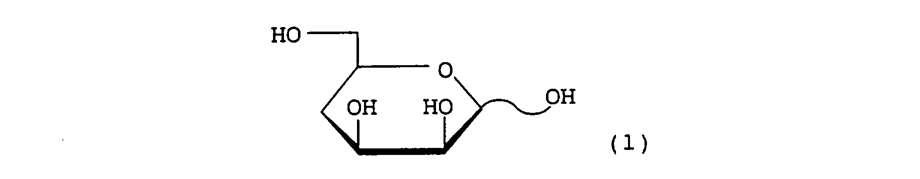

For Example, 4-deoxy-D-mannose (1) can be converted to a sialic acid derivative by use of a synthetic method or an enzyme method. The sialic acid derivative is an important sugar since it may form an active site of a sugar chain. On the other hand, a novel function may be desired to be added to the sugar chain by replacing a certain kind of monosaccharide constituting a sugar chain with 4-deoxy-D-mannose (1) itself or integrating 4-deoxy-D-mannose (1) into a sugar chain.

From the above, 4-deoxy-D-mannose (1) can be a useful raw material of a sugar chain or a compound containing a sugar, however, it can not be obtained from a natural source. Accordingly, 4-deoxy-D-mannose has been synthesized from a D-mannosan derivative through 5 processes [H. Paulsen and T. Peters, Carbohydr. Res., 165, 229 (1987)]. Further, D-mannosan has to be obtained by means of thermolysis of germ of ivory palm [A. E. Knauf, R. M. Hann, and C .S. Hudson, J. Am. Chem. Soc., 63, 1447 (1941), R. M. Hann and C. S. Hudson, J. Am. Chem. Soc., 64, 925 (1942)]. As a result, it requires 7 processes to synthesize 4-deoxy-D-mannose and an overall yield is as low as 3%.

The present invention has been developed on the basis of the above-mentioned circumstances. The object of the present invention is to provide a method of preparing 4-deoxy-D-mannose from a widely available raw material conveniently and selectively in a high yield.

The present inventors have conducted intensive and extensive studies and found an effective method of preparing 4-deoxy-D-mannose in an overall yield of as high as 52.5% by using levoglucosenone as a starting material. The novel method comprises 4 processes which is less than that of the conventional method.

That is, the present invention provides a method of preparing 4-deoxy-D-mannose represented by the following formula (1) comprising the following processes (a) to (c). 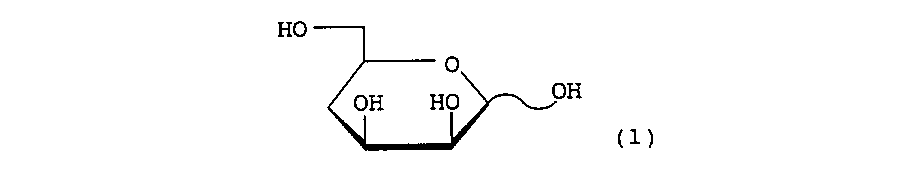 (a) a process of preparing 1,2-trans addact represented by general formula (3) by reacting a chemical compound (2) with iodine and a chemical compound containing an acyloxy ion as shown in the following reaction: 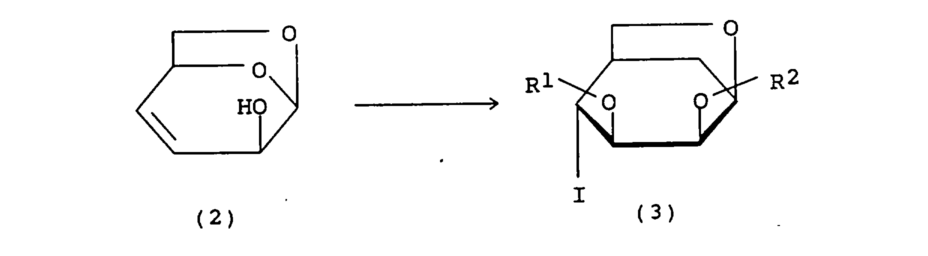 where, R¹ and R are independently a hydrogen atom or an acyl group.

(b) a process of preparing a chemical compound represented by general formula (4) by reductively removing an iodo-group of the 4-position of the chemical compound represented by general formula (3) as shown in the following reaction: 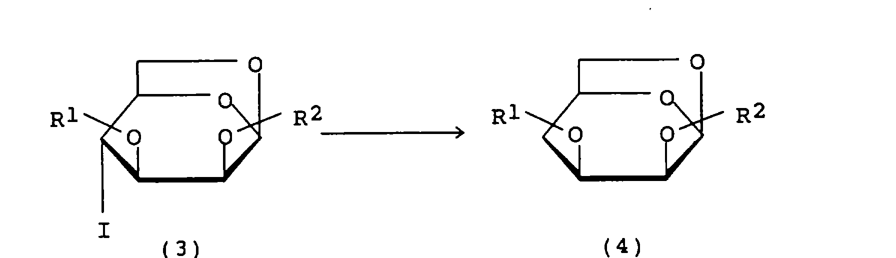 where, R¹ and R are defined above.
(c) a process of preparing 4-deoxy-D-mannose (1) by hydrolyzing a 1,6-anhydro bond and an acyl group of the compound represented by general formula (4) with an acid as shown in the following reaction:

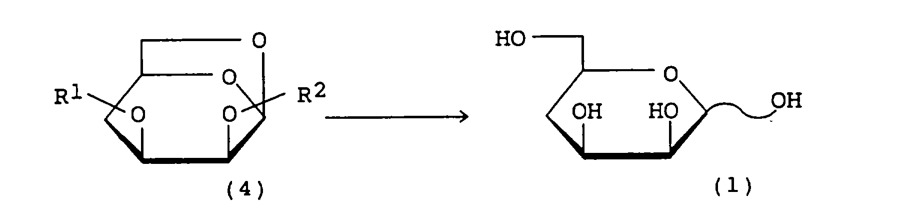

Hereinafter the method of preparing 4-deoxy-D-mannose (1) of the present invention will be described in more detail step by-step.

First, a starting raw material, 1,6-anhydro-3,4-dideoxy-β-D-threo-hex-3-enopyranose (2) can be synthesized from levoglucosenone represented by the following formula (5) according to a conventional method described in Published Unexamined Japanese Patent Application Nos. 2-272186, 3-77380 and 3-162604. That is, a carbonyl group at 2-position of levoglucosenone may be reduced by use of a reducing agent such as lithium aluminum hydride and sodium boron hydride in an appropriate solvent such as ether or tetrahydrofuran to give a hydroxyl group of a β-configuration as shown in the following reaction:

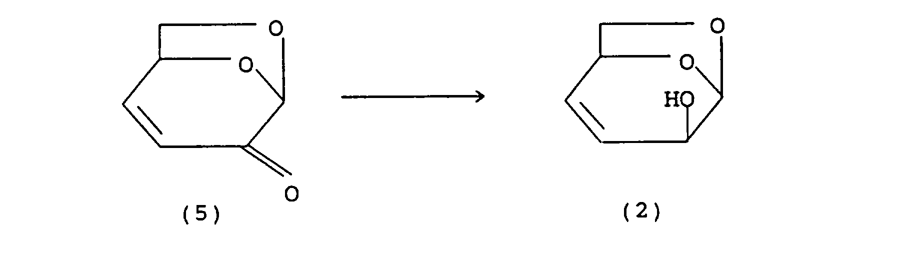

Process (a) is a process to obtain an O-acyl compound of 1,6-anhydro-4-deoxy-4-iodo-β-D-mannopyranose from 1,6-anhydro-3,4-dideoxy-β-D-threo-hex-3-enopyranose (2).

The above reaction is performed in an appropriate solvent by use of no less than 1 equivalent of iodine and no less than 1 equivalent of a carboxylic acid or a metal carboxylate relative to a double bond of the chemical compound (2).

The solvent of the above reaction is not restricted except water, alcohols, or those which release water and alcohols by hydrolysis. Preferably, a carboxylic acid, hexane, acetonitrile, and the like are used. More preferably, a carboxylic acid itself is used as a solvent. when a carboxylic acid is used as a solvent, any type of carboxylic acid can be used as long as it is a liquid state under the reaction condition. More preferably, acetic acid, propionic acid, butyric acid, and the like are used. Most preferably, acetic acid is used.

The metal carboxylate is not restricted as long as it assists to generate an iodonium ion form iodine. For example, silver acetate and cesium acetate are used. More specifically, silver acetate, silver benzoate, cesium acetate, and the like are used. The metal carboxylate is not required when a carboxylic acid is added to the reaction (including the case a carboxylic acid is used as a solvent), however, if a metal carboxylate is added thereto, a yield of a product can be improved. In the case that a carboxylic acid is not added to the reaction, a metal carboxylate is required.

The reaction temperature and the time period is not restricted, but in general, the above reaction is performed at the room temperature in a time period of between one hour and 10 days.

The chemical compound (3) obtained in this process can be a mixture of a compound (3a) in which R¹ of the 3-position is H and R of the 2-position is an acyl group and a compound (3b) in which R¹ of the 3-position is an acyl group and R of the 2-position is H. However, since the acyl group is deprotected in later step (c), the reaction can be proceeded without separating the compounds (3a) and (3b). Alternatively, a reaction can be proceeded to next step (b) after hydroxyl groups of the compounds (3a) and (3b) are acylated and purified as a 2,3-O-diacyl compound. 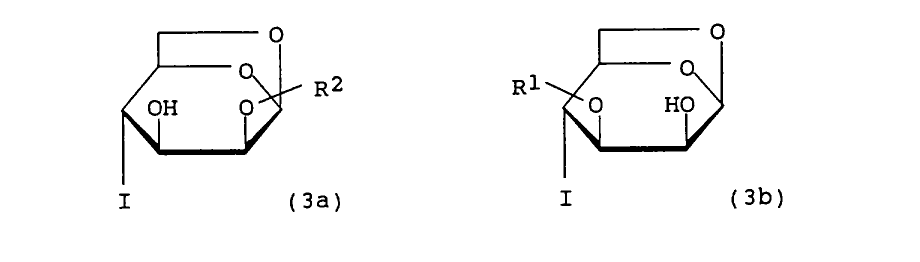 where R¹ and R are an acyl group.

The acylation is performed to treat the compounds (3a) and (3b) with an acid anhydride or an acid halide in the presence of a tertiary amine in an appropriate solvent. The acid anhydride is not restricted as long as it is used as a general acylating agent. Examples of the acid anhydride include acetic anhydride and benzoic anhydride. Also the acid halide is not restricted as long as it is used as a general acylation agent. Examples include an acid chloride such as acetyl chloride or benzoyl chloride. The solvent is not restricted as long as it is a general acylation agent. As Examples, there are mentioned a tertially amine such as pyridine and triethylamine, and a general solvent such as methylene chloride and chloroform (in this case, it is necessary to have a tertially amine in a reaction system). It is more effective if dimethylaminopyridine and the like is added to the reaction as a catalyst. The reaction temperature and the time period are not restricted. A temperature range from room temperature to 120°C and the time period of 5 minutes to 24 hours are preferable.

Process (b) is a process in which an iodo-group of the 4-position of obtained compound (3) in process (a) is radically reduced to prepare a compound (4) having no substituent at the 4-position.

The reaction in this process is performed in the presence of, for example, an organic metal hydride compound. The organic metal hydride compound is not restricted as long as it reductively removes the iodo-group. For example, tributyltin hydride, triphenyltin hydride, diphenylsilane and the like can be used. It is more effective if a compound known as a radical initiator is added to the reduction reaction as a catalyst when the organic metal hydride compound is used. As Examples of the above-mentioned compounds, there are mentioned benzoyl peroxide, α,α'-azobisisobutyronitrile (AIBN), triethylborane, and the like. An amount of the radical initiator can be varied depending on a reaction condition. Preferably, for example, the amount of 5 mol% to 20 mol% is preferable. The reaction is performed by irradiation or heating at a range of room temperature and 150°C using at least equivalent volume, more preferably, using 2 to 5 equivalent volume of the organic metal hydride compound relative to that of the compound (3). The reaction time is approximately one to 24 hours. The solvent is not specially restricted as long as it does not capture a radical, but, a hydrocarbon solvent such as benzene and toluene can be preferably used.

In process (b), when a mixture of compound (3a) and (3b) are used, a free hydroxyl group of a produced compound has to be acylated and purified as a 2,3-O-diacyl compound prior to next process (c). A method of acylation is exactly the same as that employed in process (a).

The process (c) is a process in which 1,6-anhydro bond and an acyl group of the compound represented by formula (4) obtained in process (b) is hydrolyzed in a acidic condition, thereby to obtain a desired 4-deoxy-D-mannose (1).

The solvent is preferably water or water including an appropriate organic solvent such as tetrahydrofuran - water and dioxane - water.

The acid catalyst is not specially restricted as long as a general acid which can hydrolyze a 1,6-anyhydro bond and an acyl group. The Examples include hydrochloric acid, sulfuric acid, Amberlite IR-120B^{™} (a proton type) cation ion exchange resin, and the like.

The reaction temperature is not restricted. At least room temperature or boiling temperature is preferable.

Through the above-mentioned process, 4-deoxy-D-mannose represented by formula (1) can be prepared.

### Examples

Hereinafter, the present invention will be described in detail with reference to the following Examples which should not be construed as limiting the scope of the present invention.

### Example 1

### (Process of synthesizing A raw material)

### Synthesis of 1,6-anhydo-3,4-dioxy-β-D-threo-hex-3-enopyranose

2.42g (63.8 mmol) of lithium aluminum hydride was added to 200 ml of a dry ether. 7.98g (63.3 mmol) of levoglucosenone dissolved in 130 ml of ether was added dropwise in a nitrogen atmosphere while ice cooling. Then, the reaction mixture was stirred at room temperature for one hour and 4.60g (256 mmol) of water was added dropwise. Further methanol was added thereto and filtered off insoluble material therefrom, and then a solvent was removed from the filtrate under reduced pressure. The residue was purified by means of column chromatography on silica gel (hexane : diethyl ether = 1 : 1 - 1 : 2) and recrystallized with hexane-diethyl ether mixed solvent (hexane : diethyl ether = 4 : 1), thereby obtaining 5.70g (70.3%) of 1,6-anhydro-3,4-dideoxy-β-D-threo-hex-enopyranose represented by the following formula (2). 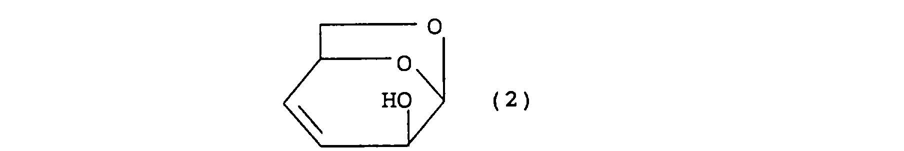
Melting point: 65.6 - 66.4°C
[α]⁵D -30.3° (c 1.00, CHCℓ₃)
IR Vₘₐₓ
   3412 (br), 3050 (w), 1425 (m), 1259 (m),
   1180 (m), 1125 (s), 1071 (s), 1046 (s)
¹H-NMR (CDCℓ₃, ppm from TMS)
   6.12 (1H, dd, J = 9.9, 4.2 Hz); the 4-position
   5.72 (1H, ddd, J = 9.9, 2.2, 2.2 Hz); the 3-position
   5.52 (1H, br); the 1-position
   4.67 (1H, dd, J = 4.2, 4.1 Hz): the 5-position
   4.34 (1H, m); the 2-position
   3.84 (1H, d, J = 6.6 Hz); 3.78 - 3.74 (1H, dd, J = 6.6, 4.1 Hz); the 6-position
   2.10 (1H, d, J = 12.0 Hz); OH.

### (Process a) Synthesis of 2-O-acetyl-1,6-anhydro-4-deoxy-4-iodo-β-D-mannopyranose and 3-O-acetyl-1,6-anhydro-4-deoxy-4-iode-β-D-mannopyranose

0.13g (1.00 mmol) of 1,6-anhydro-3,4-dideoxy-β-D-threo-hex-3-enopyranose represented by formula (2) was dissolved in 4.6 ml of acetic acid, and 0.33g (2.00 mmol) of silver acetate was added to the solution. 0.27g (1.05 mmol) of iodine was gradually added to the mixture at room temperature while vigorously stirring. After the mixture was stirred for 5 hours at room temperature under nitrogen atmosphere, 0.19g (1.27 mmol) of sodium iodide was added thereto. The reaction mixture was slowly added to an aqueous solution containing 6.96g of sodium hydrogen carbonate while ice-cooling to convert it to a neutral solution. Undissolved material was filtered off and the solvent was removed from the filtrate under reduced pressure. The residue was purified by means of column chromatography on silica gel (hexane : ethyl acetate = 1 : 3) to give 0.29g (yield:92.0%) of a mixture of 2-O-acetyl-1,6-anhydro-4-deoxy-4-iodo-β-D-mannopyranose represented by the following formula (3a) and 3-O-acetyl-1,6-anhydro-4-deoxy-4-iodo-β-D-mannopyranose represented by the following formula (3b). 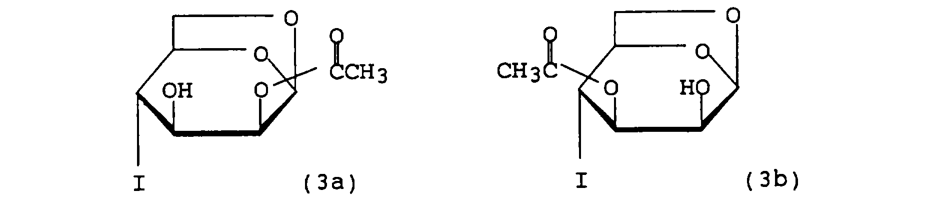
¹H-NMR (CDCℓ₃, ppm from TMS)
2-O-acetyl-1,6-anhydro-4-deoxy-4-iodo-β-D-mannopyranose (3a)
   5.53 (1H, br); the 1-position
   5.20 (1H, dd, J = 1.6, 4.8 Hz); the 2-position
   4.48 (1H, ddd, J = 1.6, 5.0, 5.0 Hz); the 3-position
   4.72 (1H, d, J = 4.8 Hz); the 5-position
   4.41-4.39 (2H, m); the 4- and the 6-position
   3.82-3.76 (1H, m); the 6'-position
   3.23 (1H, d, J = 5.3 Hz); OH
   2.18 (3H, s); OAc.
3-O-acetyl-1,6-anhydro-4-deoxy-4-iodo-β-D-mannopyranose (3b)
   5.45 (1H, br); the 1-position
   5.35 (1H, ddd, J = 1.3, 2.7, 5.5 Hz); the 3-position
   4.63 (1H, d, J = 5.5 Hz); the 5-position
   4.27 (1H, br); the 4-position
   4.20 (1H, ddd, J = 1.9, 5.5, 11.6 Hz); the 2-position
   4.11 (1H, dd, J = 0.7, 7.9 Hz); the 6-position
   3.82-3.76 (1H, m); the 6'-position
   2.39 (1H, d, J = 11.6 Hz); OH
   2.16 (3H, s); OAc.

### (process b) Synthesis of 2-O-acetyl-1,6-anhydro-4-deoxy-β-D-mannopyranose and 3-O-acetyl-1,6-anhydro-4-deoxy-β-D-mannopyranose

0.55g (1.75 mmol) of the mixture of 2-O-acetyl-1,6-anhydro-4-deoxy-4-iodo-β-D-mannopyranose (3a) and 3-O-acetyl-1,6-anhydro-4-deoxy-4-iodo-β-D-mannopyranose (3b) was dissolved in 13 ml of dry toluene, and 0.58 ml (2.16 mmol) of tributyltin hydride and catalytic amount of a,a'-azobisisobuyronitrile as a radical initiator were added thereto. After being refluxed for one hour under nitrogen atmosphere, the reaction mixture was concentrated under reduced pressure. The residue was purified by means of column chromatography on silica gel (hexane : ethyl acetate = 2 : 1 - 1 : 2) to give a mixture of 0.34g (quantitative yield) 2-O-acetyl-1,6-anhydro-4-deoxy-β-D-mannopyranose represented by following formula (4a) and 3-O-acetyl-1,6-anhydro-4-deoxy-β-D-mannopyranose represented by following formula (4b). 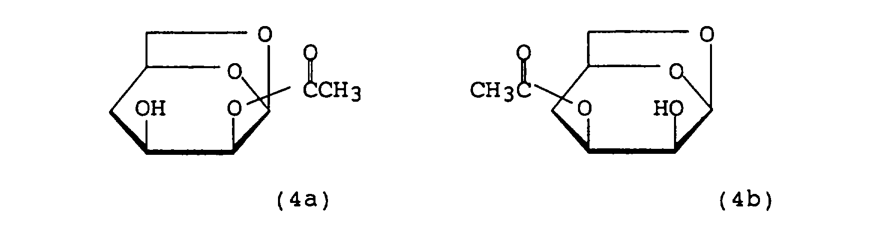
¹H-NMR (CDCℓ₃, ppm from TMS)
2-O-acetyl-1,6-anhydro-4-deoxy-β-D-mannopyranose (4a)
   5.40 (1H, br); the 1-position
   4.71 (1H, d, J = 4.7 Hz); the 2-position
   4.52 (1H, dd, J = 4.3, 4.3 Hz); the 5-position
   4.31 (1H, d, J = 6.8 Hz), 3.77-3.73 (1H, m); the 6-position
   4.21 (1H, br); the 3-position
   2.68 (1H, d, J = 4.5 Hz); OH
   2.25-2.17 (1H, m) 2.01 (1H, d, J = 15.5 Hz); 4-position
   2.14 (3H, s); OAc
3-O-acetyl-1,6-anhydro-4-deoxy-β-D-mannopyranose (4b)
   2.44 (1H, d, J = 11.3 Hz); OH
   2.10 (3H,s); OAc
   5.32 (1H, br); the 1-position
   3.67 (1H, dd, J = 5.7, 11.3 Hz); the 2-position
   5.21 (1H, br); the 3-position
   2.21 (1H, ddd, J = 4.2, 4.3, 15.8 Hz), 1.90 (1H, d, J = 15.8 Hz); the 4-position
   4.46 (1H, dd, J = 4.3, 4.4 Hz); the 5-position
   4.02 (1H, d, J = 7.0 Hz), 3.37-3.73 (1H, m); the 6-position

### (process c) Synthesis of 4-deoxy-D-mannose

0.34g of the mixture of 2-O-acetyl-1,6-anhydro-4-deoxy-β-D-mannopyranose (4a) and 3-O-acetyl-1,6-anhydro-4-deoxy-β-D-mannopyranose (4b), 10 ml of 1N hydrochloric acid and 5 ml of dioxane were combined, and the reaction mixture was heated at 100°C for 8 hours under nitrogen atmosphere. After cooling, Amberlite IRA-410™ (hydroxyl anion type) anion ion exchange resin was added to the mixture while stirring to convert it to a neutral solution. After filtration of the anion ion exchange resin, the filtrate was concentrated under reduced pressure to give 0.23g of 4-deoxy-D-mannose represented by following formula (1) [81.2% yield from compound (4)]. 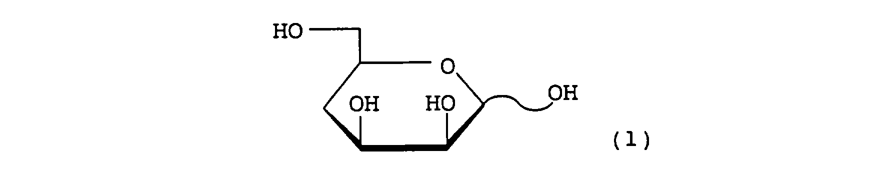
[α]⁴D +13.2° (c 1.61, CH₃OH)
¹H-NMR [D₂O, t-Butanol as a internal standard (1.23 ppm)]
   - α-pyranose:: 5.20 (1H, br); the 1-position 3.75 (1H, br); the 2-position 4.12-3.99 (2H, m); the 3- and 5-position
   1.72-1.43 (2H, m); the 4-position 3.68-3.55 (2H, m); the 6-position
   - β-pyranose:: 4.74 (1H, br); the 1-position 3.89 (1H, ddd, J = 3.0, 5.1, 12.0 Hz); the 3-position
   3.81 (1H, br); the 2-position 3.68-3.55 (3H, m); the 5- and the 6-position
¹³C-NMR [D₂O, dioxane as a internal standard (1.23 ppm)]
α-pyranose: 95.4, 69.7, 69.5, 65.5, 65.0, 29.9.
β-pyranose: 94.8, 73.4, 70.7, 68.8, 64.7, 29.3.

### Example 2

In this example, the compound (3) obtained in the process (a) was acetylated, and then the reaction after step (b) was carried out by using the acetylated compound.

### (process a) Synthesis of 2,3-O-diacetyl-1,6-anhydro-4-deoxy-4-iodo-β-D-mannopyranose

0.13g (1.00 mmol) of 1,6-anhydro-3,4-dideoxy-β-D-threo-hex-3-enopyranose (2) was dissolved in 4.6 ml of acetic acid, and 0.33g (2.00 mmol) of silver acetate was added to the solution. 0.27g (1.05 mmol) of iodine was gradually added to the mixture at room temperature while vigorously stirring. After the mixture was stirred for 5 hours at room temperature under nitrogen atmosphere, 0.19g (1.27 mmol) of sodium iodide was added thereto. The reaction mixture was slowly added to an aqueous solution containing 6.96g of sodium hydrogen carbonate while ice-cooling to convert it to a neutral solution. Undissolved material was filtered off and the solvent was removed from the filtrate under reduced pressure. The residue was purified by means of silica gel column chromatography (hexane : ethyl acetate = 1 : 3) to give a mixture of 2-O-acetyl-1,6-anhydro-4-deoxy-4-iodo-β-D-mannopyranose represented by the following formula (3a) and 3-O-acetyl-1,6-anhydro-4-deoxy-4-iodo-β-D-mannopyranose represented by the following formula (3b).

Then, the mixture of (3a) and (3b) was dissolved in 10 ml of methylene chloride, and 0.19 ml (2.01 mmol) of acetic anhydride, 0.32 ml (3.96 mmol) of pyridine and catalytic amount of N,N-dimethylaminopyridine were added thereto. After being stirred at room temperature for 30 minutes under nitrogen atmosphere, the reaction mixture was poured into ice-water. The resultant solution was extracted by methylene chloride. The organic layer was successively washed once with water, twice with an aqueous solution of copper sulfate and once with water, dried over anhydrous magnesium sulfate, and then concentrated under reduced pressure. The residue was purified by means of column chromatography on silica gel (hexane : ethyl acetate = 1 : 1) to give 0.31g [87.9% yield from compound (2)] of 2,3-O-diacetyl-1,6-anhydro-4-deoxy-4-iodo-β-D-mannopyranose represented by the following formula (3c). This product was recrystallized from hexane-diethyl ether-methylene chloride mixed solvent. 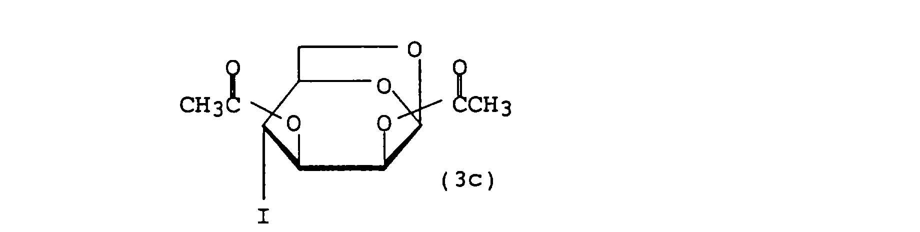
Melting point : 95.5-96.1°C
[α]⁶D -187.2° (c 1.03, CHCℓ₃)
¹H-NMR (CDCℓ₃, ppm from TMS)
   5.53 (1H, dd, J = 1.4, 5.1 Hz); the 3-position
   5.49 (1H, br); the 1-position
   5.36 (1H, dd, J = 1.6, 5.1 Hz); the 2-position
   4.69 (1H, dd, J = 0.6, 5.4 Hz); the 5-position
   4.29 (1H, dd, J = 0.6, 7.8 Hz), 3.84 (1H, dd,
   J = 5.4, 7.8 Hz); the 6-position
   4.25 (1H, br); the 4-position
   2.15 (3H, s), 2.08 (3H, s); OAc
¹³C-NMR [CDCℓ₃, ppm from CDCℓ₃ (77.40 ppm)]
   170.0 (CO of AcO), 169.8 (CO of AcO), 21.2 (CH₃ of Ac), 21.0 (CH₃ of Ac), 100.2 (C-1), 66.0 (C-2), 73.1 (C-3), 23.9 (C-4), 78.1 (C-5), 68.5 (C-6).

### (process b) Synthesis of 2,3-O-diacetyl-1,6-annhydro-4-deoxy-β-D-mannopyranose

0.71g (2.00 mmol) of 2,3-O-diacetyl-1,6-anhydro-4-deoxy-4-iodo-β-D-mannopyranose (3c) was dissolved in 17 ml of dry toluene, and 0.65 ml (2.40 mmol) of tributyltin hydride and catalytic amount of a,a'-azobisisobuyronitrile as a radical initiator were added thereto. After being refluxed for one hour under nitrogen atmosphere, the reaction mixture was concentrated under reduced pressure. Ice-water was added to the reaction mixture, and the resultant solution was extracted with methylene chloride. The organic layer was successively washed once with an aqueous solution of potassium fluoride, once with an aqueous solution of sodium hydrogen carbonate, and once with water. After removal of methylene chloride under reduced pressure, the residue was purified by means of column chromatography on silica gel (hexane : ethyl acetate = 2 : 1 - 1 : 2) to give 0.42g (90.2%)
2,3-O-diacetyl-1,6-anhydro-4-deoxy-β-D-mannopyranose represented by the following formula (4c). This product was recrystallized from hexane-diethyl ether mixed solvent. 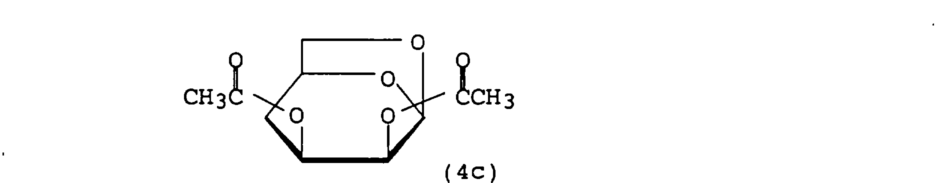
Melting point : 68.0-69.0°C
[α]⁶D -83.1° (c 0.59, CHCℓ₃)
¹H-NMR (CDCℓ₃, ppm from TMS)
   5.43 (1H, dd, J = 5.0, 5.0 Hz); the 3-position
   5.39 (1H, s); the 1-position
   4.90 (1H, dd, J = 1.7, 5.0 Hz); the 2-position
   4.57 (1H, dd, J = 4.4, 4.4 Hz); the 5-position
   4.26 (1H, d, J = 7.0 Hz), 3.85 (1H, m); the 6-position
   2.34 (1H, dddd, J = 1.7, 4.4, 5.0, 15.5 Hz),
   1.92 (1H, d, J = 15.5 Hz); the 4-position
   2.13 (3H, s), 2.07 (3H, s); OAc

### (process c) Synthesis of 4-deoxy-D-mannose

1.02g (4.41 mmol) of 2,3-O-diacetyl-1,6-anhydro-4-deoxy-β-D-mannopyranose (4c), 30 ml of IN hydrochloric acid and 15 ml of dioxane were combined, and the reaction mixture was heated at 100°C for 8 hours under nitrogen atmosphere. After cooling, Amberlite IRA-410™ (hydroxyl anion type) anion ion exchange resin was added to the mixture while stirring to convert it to a neutral solution. After filtration of the anion ion exchange resin, the filtrate was concentrated under the reduced pressure to give 0.52g (71.7%) of 4-deoxy-D-mannose represented by the following formula (1). The physical properties of the product were consistent with that of the compound (1) obtained in Example 1.

### Example 3

In this example, The reaction was carried out by using the compound which obtained by acetylation of a hydroxyl group of the compound (4) prepared from process 1⁶).

### (process b) Synthesis of 2,3-O-diacetyl-1,6-anhydro-4-deoxy-β-D-mannopyranose

0.25g (0.79 mmol) of the mixture of 2-O-acetyl-1,6-anhydro-4-deoxy-4-iodo-β-D-mannopyranose (3a) and 3-O-acetyl-1,6-anhydro-4-deoxy-4-iodo-β-D-mannopyranose (3b) obtained in process (a) in Example 2 was dissolved in 6 ml of dry toluene, and 0.26 ml (0.95 mmol) of tributyltin hydride and catalytic amount of a,a'-azobisisobuyronitrile as a radical initiator were added thereto. After the reaction mixture was refluxed for one hour under nitrogen atmosphere, a solvent was removed from the reaction solution under reduced pressure, the residue was purified by means of column chromatography on silica gel (hexane : ethyl acetate = 2 : 1 - 1 : 2) to give a mixture of 2-O-acetyl-1,6-anhydro-4-deoxy-β-D-mannopyranose (4a) and 3-O-acetyl-1,6-anhydro-4-deoxy-β-D-mannopyranose (4b).

Then, the mixture of (4a) and (4b) was dissolved in 3 ml of methylene chloride, and 0.16 ml (1.70 mmol) of acetic anhydride, 0.23 ml (1.65 mmol) of triethylamine and catalytic amount of N,N-dimethylaminopyridine were added thereto. After being stirred at room temperature for 17 hours under nitrogen atmosphere, the reaction mixture was poured into ice-water, and then extracted with chloroform. The organic layer was washed once with a diluted hydrochloric acid, once with an aqueous solution of sodium hydrogen carbonate, once with an aqueous solution of potassium fluoride, and twice with water, and then dried over anhydrous magnesium sulfate. After removal of the solvent under reduced pressure, the residue was purified by column chromatography on silica gel (hexane : ethyl acetate = 1 : 1) to give 0.17g [92.4% yield from compound (3)] of 2,3-O-diacetyl-1,6-anhydro-4-deoxy-β-D-mannopyranose (4c). This product was recrystallized from hexane-diethyl ether mixed solvent. The physical properties of the product were consistent with that of 2,3-O-diacetyl-1,6-anhydro-4-deoxy-β-D-mannopyranose (4c) obtained in Example 2.

From the above, the present invention make it possible to prepare a hard-obtaining rare sugar 4-deoxy-D-mannose in a high yield from staring levoglucosenone through a fewer processes than that of a convenient method including a reduction of a carbonyl group of levoglucosenone, an stereoselective addition reaction of an iodo-group and an acyloxy group to a double bond. Owning to this method, 4-deoxy-D-mannose used as not only a constitutional unit of useful sugar compound including a sugar chain but also as a synthetic raw material can be readily supplied.

## Claims

1. A method of preparing 4-deoxy-D-mannose represented by the following formula (1) 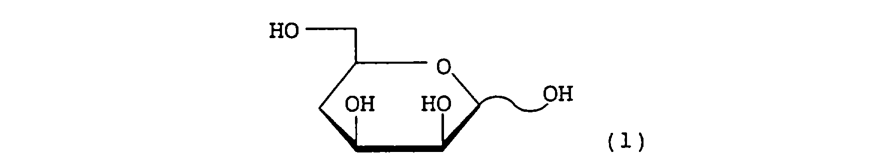 which comprises,
(a) a process of preparing a chemical compound represented by formula (3) by reacting a chemical compound represented by formula (2) with iodine and a compound containing an acyloxy ion as shown in the following reaction: 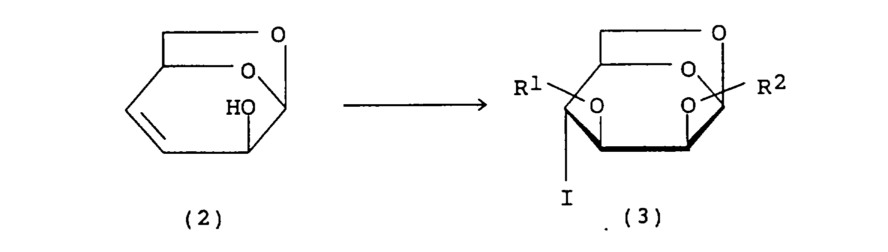 where R¹ and R are independently a hydrogen atom or an acyl group;
(b) a process of preparing a chemical compound represented by general formula (4) by reductively removing an iodo-group of the 4-position of a compound represented by general formula (3) as shown in the following reaction: 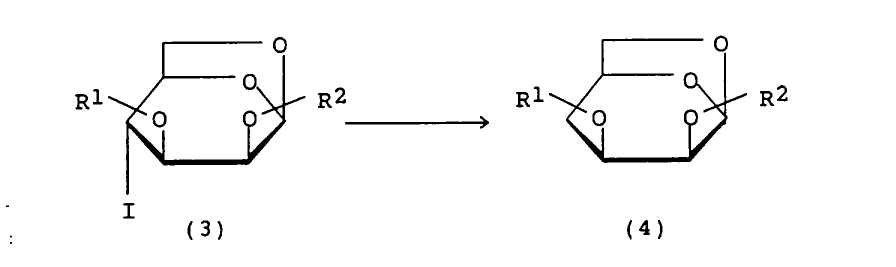 where R¹ and R are defined the above, and
(c) a process of preparing 4-deoxy-D-mannose represented by formula (1) by hydrolyzing a 1,6,-anhydro bond and an acyl group of a compound represented by formula (4) in an acidic condition as shown in the following reaction: 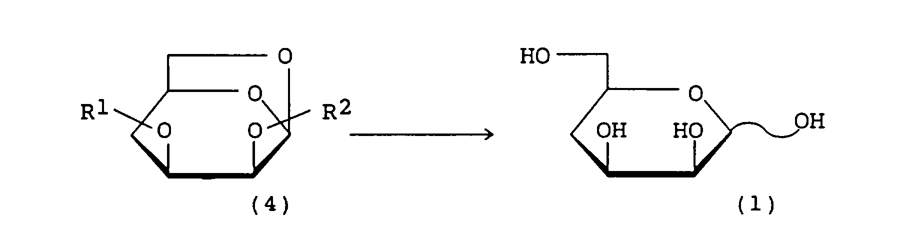 where R¹ and R are defined the above.

2. The method according to claim 1, characterized by further comprising a process of acylation and purification of a chemical compound represented by formula (3) obtained in process (a) and carrying out the reactions after said process (b) by using said acylated compound.

3. The method according to claim 1, characterized by further comprising a process of acylation and purification of a chemical compound represented by formula (4) obtained from process (b) and carrying out the reaction of said process (c) by using said acylated compound.

4. The method according to any one of claims 1 to 3, characterized in that at least one of a compound which is selected from a group consisting of a carboxylic acid and a metal carboxylate is used as said compound containing an acyloxy ion.

5. The method according to claim 4, characterized in that said carboxylic acid which is said compound containing an acyloxy ion is used as a solvent.

6. The method according to claim 4, characterized in that said carboxylic acid is selected from a group consisting of acetic acid, propionic acid, and butyric acid.

7. The method according to claim 4, characterized in that said metal carboxylate is selected from the group consisting of silver carboxylate and cesium carboxylate.

8. The method according to claim 7, characterized in that said metal carboxylate is selected from a group consisting of silver acetate, silver benzoate, and cesium acetate.

9. The method according to any one of claims 1 to 3, characterized in that said reduction reaction of process (b) is performed by use of an organic metal hydride compound in the presence or absence of a radical initiator.

10. The method according to claim 9, characterized in that said organic metal hydride compound is selected from a group consisting of tributyltin hydride, triphenyltin hydride, and diphenylsilane.

11. The method according to claim 9, characterized in that said radical initiator is selected from a group consisting of benzoyl peroxide, α,α'-azobisisobutyronitrile (AIBN), and triethylborane.

12. The method according to claims 1 to 3, characterized in that an acid used in said hydrlolysis performed in an acidic condition is selected from a group consisting of hydrochloric acid, sulfuric acid, and Amberlite IR-120B™ (a proton type) cation ion exchange resin.

## Patentansprüche

1. Verfahren zur Herstellung von 4-Desoxy-D-mannose der nachfolgenden Formel (1) 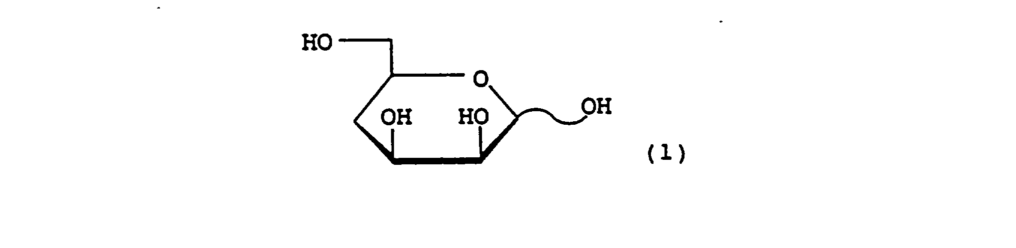 umfassend
(a) ein Verfahren zur Herstellung einer chemischen Verbindung der Formel (3) durch Umsetzen einer chemischen Verbindung der Formel (2) mit Jod und einer ein Acyloxyion enthaltenden Verbindung in der nachfolgenden Reaktion: 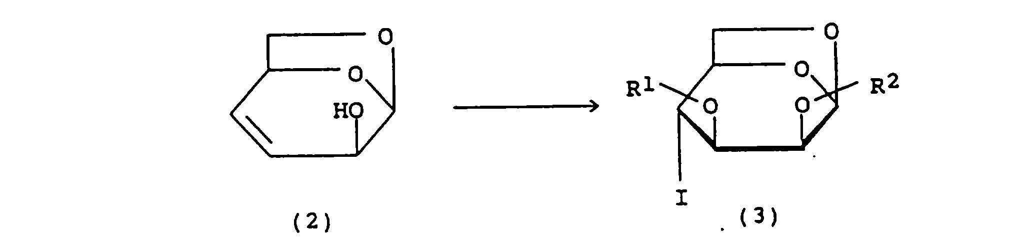 wobei R¹ und R unabhängig voneinander ein Wasserstoffatom oder eine Acylgruppe sind;
(b) ein Verfahren zur Herstellung einer chemischen Verbindung der allgemeinen Formel (4) durch reduktive Entfernung einer Jodgruppe an der 4-Position einer Verbindung der allgemeinen Formel (3) entsprechend der nachfolgenden Reaktion: 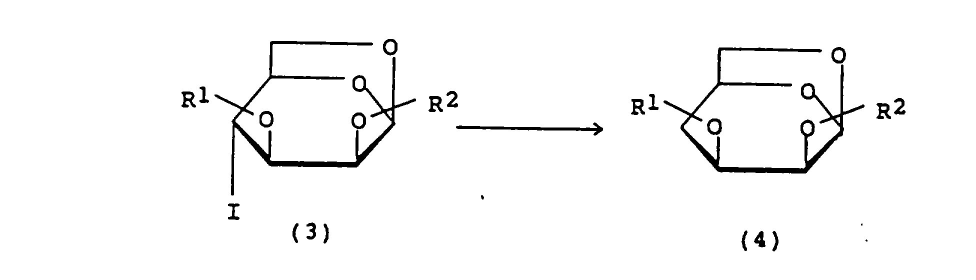 wobei R¹ und R die oben definierten Bedeutungen aufweisen, und
(c) ein Verfahren zur Herstellung von 4-Desoxy-D-mannose der Formel (1) durch Hydrolyse einer 1,6-Anhydro-Bindung und einer Acylgruppe einer Verbindung der Formel (4) im sauren Zustand entsprechend der nachfolgenden Reaktion: 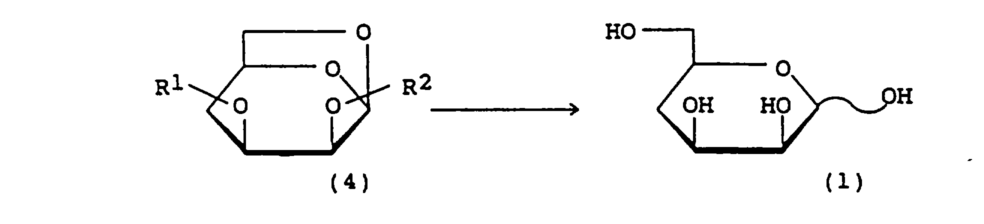 wobei R¹ und R die oben definierten Bedeutungen aufweisen.

2. Verfahren nach Anspruch 1,
dadurch gekennzeichnet,
daß es weiterhin ein Verfahren zur Acylierung und Reinigung der im Verfahren (a) erhaltenen chemischen Verbindung der Formel (3) und die Durchführung der Reaktionen nach dem Verfahren (b) unter Verwendung der acylierten Verbindung umfaßt.

3. Verfahren nach Anspruch 1,
dadurch gekennzeichnet,
daß es weiterhin ein Verfahren zur Acylierung und Reinigung der aus dem Verfahren (b) erhaltenen chemischen Verbindung der Formel (4) und die Durchführung der Reaktion des Verfahrens (c) unter Verwendung einer acylierten Verbindung umfaßt.

4. Verfahren nach einem der Ansprüche 1 bis 3,
dadurch gekennzeichnet,
daß wenigstens eine Verbindung, die aus der Gruppe, bestehend aus einer Carbonsäure und einem Metallcarboxylat ausgewählt wird, als die ein Acyloxyion enthaltende Verbindung verwendet wird.

5. Verfahren nach Anspruch 4,
dadurch gekennzeichnet,
daß die Carbonsäure, bei der es sich um die ein Acyloxyion enthaltende Verbindung handelt, als Lösungsmittel verwendet wird.

6. Verfahren nach Anspruch 4,
dadurch gekennzeichnet,
daß die Carbonsäure aus der Gruppe, bestehend aus Essigsäure, Propionsäure und Buttersäure ausgewählt wird.

7. Verfahren nach Anspruch 4,
dadurch gekennzeichnet,
daß das Metallcarboxylat aus der Gruppe, bestehend aus Silbercarboxylat und Cäsiumcarboxylat ausgewählt wird.

8. Verfahren nach Anspruch 7,
dadurch gekennzeichnet,
daß das Metallcarboxylat aus der Gruppe, bestehend aus Silberacetat, Silberbenzoat und Cäsiumacetat ausgewählt wird.

9. Verfahren nach einem der Ansprüche 1 bis 3,
dadurch gekennzeichnet,
daß die Reduktionsreaktion des Verfahrens (b) unter Verwendung einer organischen Metallhydridverbindung in Gegenwart eines Radikalinitiators oder ohne einen Radikalinitiator durchgeführt wird.

10. Verfahren nach Anspruch 9,
dadurch gekennzeichnet,
daß die organische Metallhydridverbindung aus der Gruppe, bestehend aus Tributylzinnhydrid, Triphenylzinnhydrid und Diphenylsilan ausgewählt wird.

11. Verfahren nach Anspruch 9,
dadurch gekennzeichnet,
daß der Radikalinitiator aus der Gruppe, bestehend aus Benzoylperoxid, α,α'-Azobisisobutyronitril (AIBN) und Triethylboran ausgewählt wird.

12. Verfahren nach einem der Ansprüche 1 bis 3,
dadurch gekennzeichnet,
daß die bei der in saurer Umgebung durchgeführten Hydrolyse verwendete Säure aus der Gruppe, bestehend aus Salzsäure, Schwefelsäure und Amberlite IR-120B® (ein Protontyp)-Kationenaustauschharz ausgewählt wird.

## Revendications

1. Procédé de préparation de 4-désoxy-D-mannose représenté par la formule (1) suivante 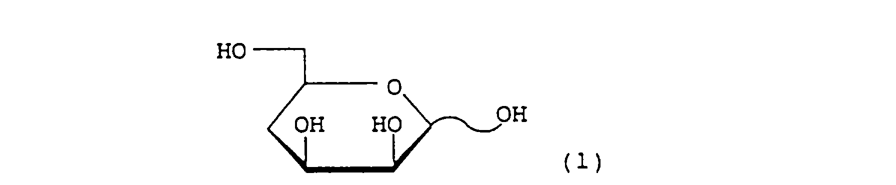 lequel comprend
(a) un procédé de préparation d'un composé chimique représenté par la formule (3) par réaction d'un composé chimique représenté par la formule (2) avec de l'iode et un composé contenant un ion acyloxy, comme représenté par la réaction suivante : 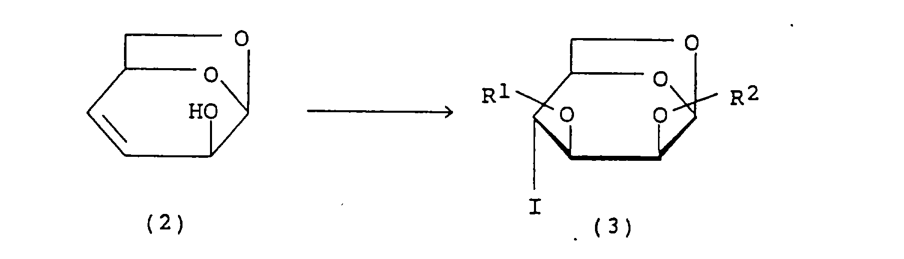 pour laquelle R¹ et R représentent indépendamment un atome d'hydrogène ou un groupement acyle ;
b) un procédé de préparation d'un composé chimique représenté par la formule générale (4) par élimination par réduction du groupement iodo en position 4 du composé représenté par la formule générale (3), comme représenté par la réaction suivante : 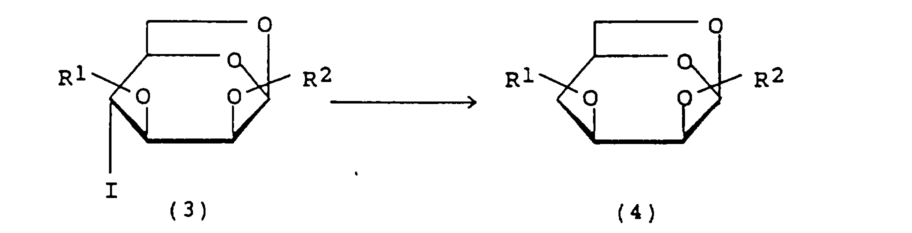 pour laquelle R¹ et R sont tels que définis ci-dessus ; et
(c) un procédé de préparation de 4-désoxy-D-mannose représenté par la formule (1) par hydrolyse de la liaison 1,6-anhydro et du groupement acyle du composé représenté par la formule (4) en condition acide, comme représenté par la réaction suivante : 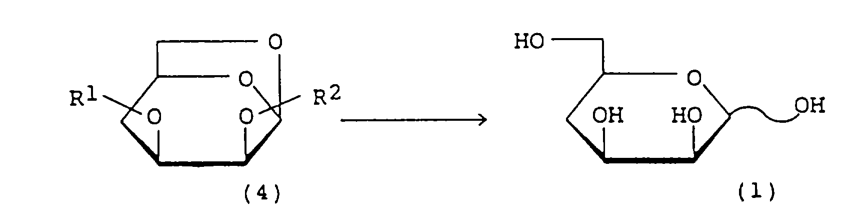 pour laquelle R¹ et R sont tels que définis ci-dessus.

2. Procédé selon la revendication 1, caractérisé en ce qu'il comprend de plus un procédé d'acylation et de purification du composé chimique représenté par la formule (3) obtenu dans le procédé (a) et en ce qu'il met en oeuvre les réactions après ledit procédé (b) en utilisant ledit composé acétylé.

3. Procédé selon la revendication 1, caractérisé en ce qu'il comprend de plus un procédé d'acylation et de purification du composé chimique représenté par la formule (4) obtenu à partir du procédé (b) et en ce qu'il met en oeuvre la réaction dudit procédé (c) en utilisant ledit composé acétylé.

4. Procédé selon l'une quelconque des revendications 1 à 3, caractérisé en ce qu'on utilise au moins un des composés choisis dans le groupe constitué d'un acide carboxylique et d'un carboxylate de métal comme ledit composé contenant un ion acyloxy.

5. Procédé selon la revendication 4, caractérisé en ce qu'on utilise ledit acide carboxylique, qui est ledit composé contenant un ion acyloxy, comme solvant.

6. Procédé selon la revendication 4, caractérisé en ce qu'on choisit ledit acide carboxylique dans le groupe constitué de l'acide acétique, de l'acide propionique et de l'acide butyrique.

7. Procédé selon la revendication 4, caractérisé en ce qu'on choisit ledit carboxylate de métal dans le groupe constitué du carboxylate d'argent et du carboxylate de césium.

8. Procédé selon la revendication 7, caractérisé en ce qu'on choisit ledit carboxylate de métal dans le groupe constitué de l'acétate d'argent, du benzoate d'argent et de l'acétate de césium.

9. Procédé selon l'une quelconque des revendications 1 à 3, caractérisé en ce qu'on réalise ladite réaction de réduction du procédé (b) en utilisant un composé de type hydrure de métal organique en présence ou en l'absence d'un imitateur de radicaux.

10. Procédé selon la revendication 9, caractérisé en ce qu'on choisit ledit composé de type hydrure de métal organique dans le groupe constitué de l'hydrure de tributylétain, de l'hydrure de triphénylétain et du diphénylsilane.

11. Procédé selon la revendication 9, caractérisé en ce qu'on choisit ledit initiateur de radicaux dans le groupe constitué du peroxyde de benzoyle, de l'α,α'-azobisisobutyronitrile (AIBN) et du triéthylborane.

12. Procédé selon les revendications 1 à 3, caractérisé en ce qu'on choisit l'acide utilisé dans ladite hydrolyse réalisée en condition acide dans le groupe constitué de l'acide chlorhydrique, de l'acide sulfurique et de la résine échangeuse d'ions cationiques (de type proton) AMBERLITE IR-120B^{®}.
